# EUROPEAN PATENT SPECIFICATION

(11) **EP 3 827 850 B1**
(45) Date of publication and mention of the grant of the patent: **10.01.2024**
(21) Application number: 20020553.2
(22) Date of filing: 23.11.2020
(51) Int. Cl.: A61L 27/34, A61L 27/36

(54) **SYSTEM AND METHOD TO USE READY PRE-PORTIONED AND PRE-PACKAGED SINGLE DOSE BONE GRAFT COMPOSITION IN GELATIN BAG OR CAPSULE AND METHOD OF PRODUCING IT**
SYSTEM UND VERFAHREN ZUR VERWENDUNG EINER FERTIGEN VORPORTIONIERTEN UND VORVERPACKTEN EINZELDOSIS-KNOCHENTRANSPLANTATZUSAMMENSETZUNG IN GELATINEBEUTELN ODER -KAPSELN UND VERFAHREN ZU IHRER HERSTELLUNG
SYSTÈME ET PROCÉDÉ PERMETTANT D'UTILISER UNE COMPOSITION DE GREFFE OSSEUSE UNIDOSE PRE-PORTIONNÉE ET PRE-CONDITIONNÉE DANS UN SAC OU UNE CAPSULE DE GÉLATINE ET SON PROCÉDÉ DE PRODUCTION

(30) Priority: 27.11.2019 US 201916602715
(43) Date of publication of application: 02.06.2021
(73) Proprietor: Roshkovan, Igor, Los Angeles, CA 90035 (US)
(72) Inventor: Roshkovan, Igor, Los Angeles, CA 90035 (US)
(74) Representative: Margue, Robert Germain

(56) References cited:
- WO-A2-2007/002085
- WO-A2-2013/192138
- JP-A- H07 246 235

## Description

### BACKGROUND - PRIOR ART

This disclosure concerns improved dental care for partially edentulous or fully edentulous patients, and more to a novel system and method to use ready pre-portioned and pre-packaged single dose bone graft composition in gelatin bag or encapsulated by gelatin film of performing any bone augmentation surgery and method of producing it.

This inventions herein refer to a bone graft, to a technique of making the pre-portioned and pre-packed bone graft single dose composition in gelatin bag.

More specifically, these inventions relate to a new system and approach using portioned bone graft composition in a gelatin bag and a method of producing it.

In addition, the current invention related to bone graft composition single dose encapsulation or bone graft composition single dose microencapsulation or bone graft composition nanoencapsulation.

Bone grafting augmentation is a surgical procedure that replaces absent bone matrix with material from patient's own body, an artificial or synthetic and natural substitute or mix of it.

Bone grafts have been predominantly used to treat bone defects or bone loss or bone damage.

Bone grafting surgical procedure is used because bone tissue has the ability to regenerate partially or completely. As natural bone grows, it mostly replaces the bone grafting material, resulting in a fully integrated augmented area by new bone.

The bone graft augmentation procedure is used to increase the volume of bone and allow residual bone to accommodate functional dental implants in the atrophic area of the maxilla or mandibula jaws.

Bone augmentation or bone repair is a surgical procedure that uses particulate bone grafts or bone substitutes, or mixtures thereof cover by barrier membrane. Typical up-to-date procedures employ a barrier material which is applied over the bone graft.

Moreover, a health professionals may have some complications, and during bone augmentation surgeries the volume of bone augmentation in surgery is difficult to control by a dental provider and causes pain and discomfort for the patient after a surgical procedure and causes a list of postoperative complications.

The following is a tabulation of some prior art that presently appears relevant:

### U.S. Patents

| **Patent Number** | **Kind Code** | **Issue Date** | **Patentee** |
|---|---|---|---|
| 5711315 | A | 1998-01-27 | Jerusalmy |
| 7125253 | B2 | 2006-10-24 | Kitamura et al. |

### U.S. Patent Application Publications

| **Patent Number** | **Kind Code** | **Issue Date** | **Patentee** |
|---|---|---|---|
| 20050021142 | A1 | 2005-01-27 | Ganz et al. |
| 20080161934 | A1 | 2008-07-03 | Yamada |
| 20100291511 | A1 | 2010-11-18 | Lee |

Many different type of bone materials uses as a bone graft substance such autologous or autogenous bone grafting, allogeneic grafts, xenograft, synthetic substitutes: alloplastic grafts may be made from hydroxyapatites.

Bone graft materials are considered for bone tissue regeneration, osteoconduction, hardness of bone and acceptability by human bone.

An addition, in implant dentistry resorbable and nonresorbable membrane have been used by dental practitioners for over 20 years as barrier membranes.

The resorbable collagen membrane is a well-established biomaterial which used in alveolar ridge augmentation with maxillary sinus elevation surgery (Zitzmann NU, Naef R, Schärer P., "Resorbable versus nonresorbable membranes in combination with Bio-Oss for guided bone regeneration." Int J Oral Maxillofac Implants 1998 Jul-Aug;13(4):576.; Silva LD, de Lima VN2, Faverani LP, de Mendonga MR, Okamoto R, Pellizzer EP., "Maxillary sinus lift surgery-with or without graft material? A systematic review." Int J Oral Maxillofac Surg. 2016 Dec; 45(12):1570-1576; Wallace SS, Froum SJ. "Effect of maxillary sinus augmentation on the survival of endosseous dental implants. A systematic review," Ann Periodontal. 2003 Dec;8(1):328-43; Esposito M, Grusovin MG, Rees J, Karasoulos D, Felice P, Alissa R, Worthington H, Coulthard P. "Effectiveness of sinus lift procedures for dental implant rehabilitation: a Cochrane systematic review," Eur J Oral Implantol. 2010 Spring;3(1):7-26.; Esposito M, Felice P, Worthington HV. "Interventions for replacing missing teeth: augmentation procedures of the maxillary sinus," Cochrane Database Syst Rev. 2014 May 13; (5)).

A dental surgeon may face some intraoperative and postoperative complications during augmentation surgeries such as sinus or Schneiderian membrane perforation during graft placement, bone graft exposure, postoperative incidences of dislodgement of the bone graft and loss of bone graft (Al-Dajani M. "Recent Trends in Sinus Lift Surgery and Their Clinical Implications," Clin Implant Dent Relat Res. 2016 Feb;18(1):204-12; Ohayon L, Taschieri S, Friedmann A, Del Fabbro M. "Bone Graft Displacement After Maxillary Sinus Floor Augmentation With or Without Covering Barrier Membrane: A Retrospective Computed Tomographic Image Evaluation," Int J Oral Maxillofac Implants. 2018 Dec 5; Al-Dajani M. "Incidence, Risk Factors, and Complications of Schneiderian Membrane Perforation in Sinus Lift Surgery: A Meta-Analysis," Implant Dent. 2016 Jun; 25(3):409-15).

WO 2007/002085 A2 (PEZESHKIAN ALEX AHMAD [US]) 4 January 2007 discloses a surgical dental graft appliance (bone augmentator) comprising a vessel made of a resorbable material e.g. collagen filled with a granularized bone augmentation material e.g. bone granules.

There are a potential surgical complications that occur using barrier membrane such as exposure bone material, and difficulty in handling bone graft particles or chips during surgical procedure.

The particles or chips of bone graft coherent mass have a propensity to detach from bone graft carrier during the delivery to surgery site or stick on surface of bone graft carrier.

The scope of protection is defined by the claims. Any "embodiment" or "example" which is disclosed in the description but is not covered by the claims should be considered as presented for illustrative purpose only. Embodiments in the description relating to methods of treatment are not covered by the claims.

The present invention relates to a pre-portioned and pre-packaged single dose bone graft composition according to claims 1-7 and to the method to make it according to claims 8-11.

The pre-proportioned and pre-packaged single dose bone graft is destined to make bone augmentation surgeries easy for a dental surgeon with minimum numbers of complications.

Gelatin is a polypeptide with molecular weight between 15,000 and 249,000 g/mole, which is producing by separating chemicals elements of animal skin and bone collagen.

According to the invention the portioned bone graft composition is covered by blood-soluble or saliva-soluble film from gelatin.

### BRIEF SUMMARY AND OBJECTS OF EMBODIMENT

In reaction to the complications and problems discussed herein, and the present inventions are intended to propose a system and method that is very effective with less complications using a pre-portioned and ready pre-packaged single dose bone graft composition in gelatin bag or encapsulated by gelatin film and method to make it is provided.

An addition, encapsulation of bone graft material relates to process which enable to formulate one or more active compounds, inside modified bone graft particles with a specific properties.

An advanced aspect of the current inventions is that the single dose bone graft in gelatin bag prevents the displacement bone graft particles or ships from adhering to the walls of gelatin bags and increase of bone graft particles.

According to a further aspect of the present invention bone graft particles are an active compound in the encapsulation material.

An additional aspect of present invention that bone graft particles used as major **core** material or matrix core with optimal consolidation.

The present method can be used to enhance the stability or immobilized of the bone graft is to tightly pack the graft particles.

The invention related to pre-portioned and ready pre-packaged single dose bone graft composition in gelatin bags and method of producing it.

A single dose of the bone graft composition is placed in bags of blood-soluble gelatin film, preferably made from bone graft chip size by granulation.

In the context of one embodiment the granulation implicates any shaping process which is indicated to particles of predefined size.

Also disclosed is a method of producing gelatin bags for coating pre-portioned bone graft.

Moreover, an individual portion or individual dose or single dose of bone graft modeling by a process called press agglomeration, that is compression bone particles.

An addition, the bone graft particles have different sizes and mixtures comprising different proportion, weight of small and large sizes of bone particles. Particle sizes ranged from a mean of 150-400 microns (µm) as a small size to a large size of up to 1559 µm.

According to further aspect the bags contain blood-soluble gelatin or gelatinous substances film.

Moreover, gelatin bags will be prepared, pre-portioned and can used by dental surgeon or any other medical provider.

The gelatin blood-soluble film bag has thickness preferably up to 50 µm.

The present inventions can be widely used in other medical fields such as dentistry, orthopedic surgery, spine surgery, plastic and reconstruction surgery, sport medicine, trauma surgery, and veterinary.

The foregoing advantages and objectives of the present embodiment will be more fully understood upon consideration of the following detailed description of the present invention.

### DETAILED DESCRIPTION OF THE PREFERRED EMBODIMENT

The present invention describes a method to use ready pre-portioned and pre-packaged single dose bone graft composition in gelatin film and its derivatives bags or capsules in dental surgeries and method of producing it.

The system and method described in present invention developed new approach of system and method of alveolar ridge augmentation using a pre-portioned and ready pre-packaged single dose bone graft composition in gelatin bags.

The mass of bone graft consists of a different variety of sizes of bone chips or particles and use as active compound of major core material or matrix core.

The coherent mass of bone graft chips can harvest from cortical, cancellous or mix of both types of bone.

An addition, many different type of bone materials can use as a bone graft substance such autologous or autogenous bone grafting, allogeneic grafts, xenograft, synthetic substitutes: alloplastic grafts may be made from hydroxyapatites.

The bone graft can position in the newly formed bone cavity to preserve the volume of bone, and the space can fill with the single doses of bone graft in the bags of blood-soluble and saliva-soluble gelatin film.

Method that can be used to enhance the stability of bone graft for instance, to tightly pack of the graft particles.

Thereby, the above-mentioned discussion discloses and describes simply exemplary embodiments of the present inventions.

Hereby, an individual portion or individual dose of bone graft can be easily dosed and/or mixed with different types of bone graft materials by directly placing the dose in the bags or capsules from gelatin.

Moreover, alginates can be use as encapsulation materials for bags.

Preferably, the process of gelatin preparation starts from blending and heating granulated gelatin and other components in heated warm water in a gelatin melting reservoir. With correct heat level, mixing and vacuum, the components form thick mass combination for use in encapsulation process.

Some other ingredients can be added during the melting process such as surfactants, also wetting agents, emulsifying agents, suspending agents.

Moreover, the bone graft preparation or the process of preparing the fill material will be encapsulated by gelatin.

As an encapsulation or the process of transforming the gel mass into a thin layer of gelatin film and wrapping it around the bone grafts forms a pre-portioned and pre-packaged bone graft in gelatin bags.

Preferably, processing reservoirs, mixer apparatus, vacuum homogenizers, sieves and mills, heated and unheated transfer containers, separators may be used for the fill bone graft and gelatin while waiting for encapsulation and can be used to make a portioned bone graft composition in blood-soluble gelatin film bag.

The next step in this process is the drying stage or process which eliminates excess moisture from the gelatin film to shrink and firm up the gelatin bag. Drying occurs by a combination of tumbling and drying.

The bottom gelatin film covers the metal tray with prefabricated concave mold type unit doses, after filing these mold units with bone graft materials a second top gelatin film applied on the top surface of the filled mold type unit doses metal tray.

Afterwards sealing and cutting equipment elements are deployed to create a form of pre-portioned and pre-packaged single dose bone graft in gelatin bags.

An addition, the single dose in the bags of blood-soluble gelatin film is preferably heat-sealed by permanently heated sealing element or apparatus.

A further aspect of the present invention, other encapsulation processes can be using such as dripping or gelation method, spray-drying method, spray-cooling method, coating process, and emulsion method.

A spin disk encapsulation apparatus for forming microencapsulated transplant containing biological tissue such as bone graft coated by gelatin.

Preferably, the dripping method is based on the mechanism of droplet elongation using gravity force using single, multiple, or concentric nozzles of encapsulating apparatus.

The next step in dripping method is to apply the jet braker plate with vibrating function and spinning cup device adjustably rotatable about its main axis for forming droplets.

In other aspect the biopolymer blend coated particles are propelled upward by the centrifugal force from the mixing chamber along the inner surface of the cup into the gelling solution in one of the selected gelation bath or in an ambient or cold air.

Using this dripping method, the encapsulation particles size of bone graft composition will be up to 7-8 mm.

In addition, the final wet stage of capsules can be dried.

The present inventions can be widely used in other medical fields such as dentistry, orthopedic surgery, spine surgery, plastic and reconstruction surgery, sport medicine, trauma surgery, phinoplasty surgery and veterinary.

## Claims

1. Pre-portioned and pre-packaged single dose bone graft composition encapsulated in a bag or capsule, wherein said bag or capsule is made of gelatin, and **characterised in that** the said bag or capsule has a film thickness of up to 50 µm.

2. Pre-portioned and pre-packaged single dose bone graft composition according to claim 1, consisting of a different variety of sizes of bone chips or particles and comprises coherent mass bone graft chips harvested from cortical human bone, cancellous human bone, and mixtures thereof or an artificial or synthetic bone graft particles or transplanted from different species.

3. Pre-portioned and pre-packaged single dose bone graft composition according to claim 1 or 2, wherein said bone graft composition uses as active compound of major core material or matrix core and bone particle sizes have ranged from a mean of 150-400 microns (mu) as a small size to large size up to 1559 mu.

4. Pre-portioned and pre-packaged single dose bone graft composition according to claim 2 or 3, wherein nonsynthetic bone graft chips consist of completely demineralized or partially demineralized bone particles or mixtures thereof.

5. Pre-portioned and pre-packaged single dose bone graft composition according to any one of claims 2 to 4, wherein said bone graft chips be used in powder, particle, and mix crushed forms.

6. Pre-portioned and pre-packaged single dose bone graft composition according to any one of claims 2 to 5, wherein said that bone graft chips be used in moist or cryodesiccation forms and dry form.

7. Pre-portioned and pre-packaged single dose bone graft composition according to any one of the previous claims, wherein said bag or capsules further comprises a collagen, or gelatin, or alginate, or starch, or glycogen, or cellulose or other products.

8. Method of making a pre-portioned and pre-packaged single dose bone graft composition of any one of the previous claims, comprising the steps of
(a) providing a mixed individual dose with different types of bone graft materials;
(b) directly placing bone graft material on a gelatin film; and
(c) heat-sealing the individual dose of bone graft in gelatin film bag.

9. Method according to claim 8, wherein said that the further comprising modeling the bone graft materials composition by press agglomeration.

10. Method of claim 8 or 9, further comprising preparing individual dose of the bone graft using equipment including processing reservoirs, mixer tanks, mills, vacuum homogenizers, sieves, to dry the sealed bag to remove excess humidity, to shrink and stabilize the gelatin bags.

11. The method of any one of claims 8 to 10, wherein different methods are used for forming bone graft bags or capsules or microcapsules such as dripping or gelation method, spray-drying method, spray-cooling method, coating process, and emulsion method to encapsulate bone graft composition.

## Patentansprüche

1. Vorportionierte und vorverpackte Einzeldosis-Knochenspanzusammensetzung, eingekapselt in einem Beutel oder einer Kapsel, wobei der Beutel oder die Kapsel aus Gelatine besteht, und **dadurch gekennzeichnet, dass** der Beutel oder die Kapsel eine Filmstärke von bis zu 50 µm hat.

2. Vorportionierte und vorverpackte Einzeldosis-Knochenspanzusammensetzung gemäß Anspruch 1, bestehend aus einer unterschiedlichen Auswahl von Grö-ßen von Knochensplittern oder -teilchen, und umfassend Knochenspansplitter kohärenter Masse, gewonnen aus menschlicher Kortikalis, menschlicher Spongiosa, und Gemischen davon, oder künstliche oder synthetische Knochenspanteilchen oder transplantiert von anderen Arten.

3. Vorportionierte und vorverpackte Einzeldosis-Knochenspanzusammensetzung gemäß Anspruch 1 oder 2, wobei die Knochenspanzusammensetzung als aktive Verbindung als größeres Kernmaterial oder Matrixkern nutzt, und wobei Knochenpartikel Größen haben im Bereich von 100 bis 400 Mikrometer als kleine Größe bis zu einer großen Größe von 1559 Mikrometer.

4. Vorportionierte und vorverpackte Einzeldosis-Knochenspanzusammensetzung gemäß Anspruch 2 oder 3, wobei nicht-synthetische Knochenspanchips bestehen aus vollständig demineralisierten oder teilweise demineralisierten Knochenpartikeln oder deren Gemischen.

5. Vorportionierte und vorverpackte Einzeldosis-Knochenspanzusammensetzung gemäß irgendeinem der Ansprüche 2 bis 4, wobei die Knochenspanchips in Pulver-, Teilchen- und mischzerkleinerter Form benutzt werden.

6. Vorportionierte und vorverpackte Einzeldosis-Knochenspanzusammensetzung gemäß irgendeinem der Ansprüche 2 bis 5, wobei die Knochenspanchips in feuchter oder gefriergetrockneter Form, oder in trockner Form benutzt werden.

7. Vorportionierte und vorverpackte Einzeldosis-Knochenspanzusammensetzung gemäß irgendeinem der vorhergehenden Ansprüche, wobei der Beutel oder die Kapseln zudem ein Collagen, oder Gelatine, oder Alginat, oder Stärke, oder Glykogen, oder Cellulose oder andere Produkte umfasst.

8. Herstellungsverfahren für eine vorportionierte und vorverpackte Einzeldosis-Knochenspanzusammensetzung aus irgendeinem der vorhergehenden Ansprüche, umfassend die Schritte
(a) Bereitstellen einer gemischten Einzeldosis mit unterschiedlichen Typen Knochenspanmaterialien;
(b) direktes Platzieren von Knochenspanmaterial auf einen Gelatinefilm; und
(c) Heißsiegeln der Einzeldosis Knochenspan in einen Gelatinefilmbeutel.

9. Verfahren gemäß Anspruch 8, wobei das zudem umfasst Modellieren der Knochenspanmaterial-Zusammensetzung durch Pressagglomeration.

10. Verfahren gemäß Anspruch 8 oder 9, zudem umfassend Herstellen einer Einzeldosis des Knochenspans mit Hilfe von Ausstattung, einschließlich Verarbeitungsbehältern, Mischtanks, Mühlen, Vakuumhomogenisierern, Sieben, zum Trocknen des versiegelten Beutels zum Entfernen überschüssiger Feuchtigkeit, zum Schrumpfen und Stabilisieren der Gelatinebeutel.

11. Verfahren gemäß irgendeinem der Ansprüche 8 bis 10, wobei unterscheidliche Verfahren verwendet werden für die Bildung der Knochenspanbeutel oder -kapseln oder -mikrokapseln, wie Tropf- oder Gelierungsverfahren, Sprühtrocknungsverfahren, Sprühkühlverfahren, Beschichtungsprozesse, und Emulsionsverfahren zum Einkapseln der Knochenspanzusammensetzung.

## Revendications

1. Composition de greffe osseuse en dose unique préportionnée et préemballée, encapsulée dans un sac ou une capsule, où ledit sac ou ladite capsule est fait de gélatine, et **caractérisée en ce que** ledit sac ou ladite capsule a une épaisseur de filme de jusqu'à 50 µm.

2. Composition de greffe osseuse en dose unique préportionnée et préemballée selon la revendication 1, constitué en und variété différente de tailles de copeaux ou de particules d'os et comprenant une masse cohérente de copeaux de greffe osseuse récoltée d'os cortical humain, d'os spongieux humain, et de leurs mélanges, ou de particules de greffe osseuse artificielle ou synthétique ou transplantés d'espèces différentes.

3. Composition de greffe osseuse en dose unique préportionnée et préemballée selon la revendication 1 ou 2, où ladite composition de greffe osseuse utilise comme composé actif de matériau-noyau majeur ou matrice-noyau et des particules osseuses ayant des tailles dans un intervalle d#une moyenne de 150 à 400 microns (mu) comme petite taille à und grande taille jusqu'à 1559 mu.

4. Composition de greffe osseuse en dose unique préportionnée et préemballée selon la revendication 2 ou 3, dans laquelle des copeaux de greffe osseuse non-synthétiques consistent en des particules osseuses complètement déminéralisées ou partiellement déminéralisées ou de leurs mélanges.

5. Composition de greffe osseuse en dose unique préportionnée et préemballée selon la revendication 2 à 4, dans laquelle lesdits copeaux de greffe sont utilisés en formes de poudre, de particules ou de mélange concassé.

6. Composition de greffe osseuse en dose unique préportionnée et préemballée selon la revendication 2 à 5, dans laquelle lesdits copeaux de greffe sont utilisés en formes humide ou lyophilisée ou en forme sèche.

7. Composition de greffe osseuse en dose unique préportionnée et préemballée selon une quelconque des revendications précédentes, dans laquelle ledit sac ou lesdites capsules comprennent en plus un collagène, ou de la gélatine, ou de l'alginate, ou de l'amidon, ou du glycogène, ou de la cellulose, ou d'autres produits.

8. Procédé de production d'une composition de greffe osseuse en dose unique préportionnée et préemballée d'une quelconque des revendications précédentes, comprenant les étapes de
(a) fournir une dose individuelle m#elang#ee avec différents types de matériaux de greffe osseuse ;
(b) placer directement du matériau de greffe osseuse sur un film de gélatine ; et
(c) sceller à chaud la dose individuelle de greffe osseuse dans un sac de gélatine.

9. Procédé selon la revendication 8, dans lequel ledit comprend en plus modéliser la composition de matériaux de greffe osseuse par presse à agglomérer.

10. Procédé selon la revendication 8 ou 9, comprenant en plus préparer une dose individuelle de greffe osseuse en utilisant de l'équipement y compris des réservoirs de traitement, des mélangeurs, des broyeurs, des homogénéisateurs à vide, des tamis, pour sécher le sac scellé pour enlever de l'humidité en excès, pour contracter et stabiliser les sacs de gélatine.

11. Procédé selon une quelconque des revendications 8 à 10, dans lequel différentes méthodes sont utilisées pour former des sacs ou capsules ou microcapsules de greffe osseuse, comme des méthodes de dégouttement ou de gélation, des méthodes de séchage par atomisation, des méthodes de refroidissement par atomisation, de procédés de revêtement, de méthodes d'émulsion pour encapsuler une composition de greffe osseuse.
